# EUROPÄISCHE PATENTSCHRIFT

(11) **EP 2 165 696 B1**
(45) Veröffentlichungstag und Bekanntmachung des Hinweises auf die Patenterteilung: **09.04.2014**
(21) Anmeldenummer: 09167969.6
(22) Anmeldetag: 17.08.2009
(51) Int. Cl.: A61K 8/37, A61Q 15/00, A61Q 17/04

(54) **Kosmetische und dermatologische Formulierungen enthaltend Phenoxyalkylester**
Cosmetic and dermatological formulations containing phenoxyalkyl esters
Formules cosmétiques et dermatologiques comprenant de l'ester de phénoxyalkyle.

(30) Priorität: 17.09.2008 DE 102008042149
(43) Veröffentlichungstag der Anmeldung: 24.03.2010
(73) Patentinhaber: Evonik Degussa GmbH, 45128 Essen (DE)
(72) Erfinder: Springer, Oliver, 46485, Wesel (DE); Jenni, Klaus, 45357, Essen (DE)
(74) Vertreter: Lang, Arne

(56) Entgegenhaltungen:
- EP-A- 1 588 693
- WO-A-00/68176
- WO-A-03/032919
- WO-A-2007/087278
- DE-A1- 2 519 433
- US-A- 1 804 503

## Beschreibung

### Gebiet der Erfindung

Die vorliegende Erfindung betrifft die Verwendung von Phenoxyalkylestern von C6-C12 Alkyl- und Cycloalkylcarbonsäuren zur Herstellung einer kosmetischen, dermatologischen oder pharmazeutischen Formulierung. Die Phenoxyalkylester verleihen den Formulierungen gute sensorische Eigenschaften und zeichnen sich durch sehr gute Lösungseigenschaften für **aktive** Substanzen, insbesondere organische UV-Lichtschutzfilter und Deodorant- oder Antitranspirantwirkstoffen aus.

### Stand der Technik

Im Bereich kosmetischer Emulsionen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die hohen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet.

Weiterhin ist bekannt, dass insbesondere der ultraviolette Anteil von natürlichen und künstlichen Lichtquellen (UV-A 320-390 nm; UV-B 280-320 nm; UV-C 100 oder 200-280 nm) in größeren Mengen zur Schädigung der menschlichen Haut führt.

Die UV-A Strahlung bewirkt hauptsächlich die Hautalterung (Oberhautverdünnung und Bindegewebedegeneration, Pigmentstörungen), während UV-B und UV-C zu Sonnenbrand und Hautkrebs führen.

Das insbesondere in den letzen Jahren veränderte Freizeitverhalten mit verlängerten Aufenthalten im Freien und insbesondere extensives Sonnenbaden zur Erzielung der "gesunden Bräune" haben allerdings vor dem Hintergrund der medizinischen Erkenntnisse, des Bewusstseins der mangelnden natürlichen Schutzmechanismen der Haut durch Pigmentbildung und Sonnengewöhnung durch Verdickung der Hornschicht, die Notwendigkeit eines ausreichenden Schutzes vor intensiver UV-Strahlung weit in den Vordergrund gerückt. Deutlich verstärkt wurde sie durch die Diskussion der Abnahme und Verdünnung der antarktischen Ozonschicht und der damit einhergehenden Steigerung der Intensität der UV-A und UV-B Strahlung auf der Erdoberfläche.

Das wird deutlich an den in den letzten Jahren steigenden Umsätzen von Produkten mit hohen Sonnenschutzfaktoren (sun protection factor: SPF). Dies sind in erster Linie immer noch die klassischen Sonnenschutzformulierungen (Sonnenmilch, Sonnenöl) mit dem primären Anwendungszweck Sonnenbaden, jedoch zunehmend auch die sogenannten Pflegeprodukte für Gesicht, Körper und Haar wie Tages- und Nachtcremes, Conditioner, Lotionen, (Hydro-, Lipo)Gelen, (Lippen-)Stiften und Sprays, pharmazeutische Formulierungen und in geringem Umfang Produkte der dekorativen Kosmetik, die überwiegend in Form von Ölen und flüssigen, cremeförmigen oder salben/pastenartigen W/O- und O/W- Emulsionen im Handel sind.

Der Lichtschutzfaktor LSF oder auch SPF ist ein Koeffizient, welcher das Vermögen eines Produktes ausdrückt, Sonnenbrand durch die Sonne zu verhindern. Lichtschutz mit einem Faktor von 60 schützt daher doppelt so lange wie ein Produkt mit Faktor 30 und entsprechend dreimal solange wie ein Produkt mit Faktor 20 vor dem Auftreten eines Sonnenbrandes.

Diese höheren Lichtschutzfaktoren werden in den meisten Fällen erzeugt durch eine Erhöhung der Konzentration von UV-Lichtschutzfiltern in der Formulierung.

Seit 1995 werden die Lichtschutzfaktoren nach derselben internationalen Norm (COLIPA) gemessen, die einen Vergleich zwischen den Produkten der verschiedenen Hersteller erlaubt.

Bei den häufigen und großflächigen Anwendungen ist es nicht ausgeschlossen, dass die hochdosierten Filter (ca. 3 bis 30 Gew.-% der Formulierung) in Gramm-Mengen auf die Haut aufgetragen werden. Diese Mengen an Filtersubstanzen müssen aber gelöst und homogen und stabil in der Formulierung eingearbeitet worden sein.

Zum Lösen dieser Substanzen wird häufig von ölartigen Komponenten Gebrauch gemacht, die ein gutes Lösungsvermögen für die Filtersubstanzen besitzen. So werden unter anderem auch bestimmte Esteröle eingesetzt. Eine verwendete Verbindungsklasse sind hier aliphatische Benzoesäureester. Ein typischer Vertreter dieser Verbindungsklasse ist die Verbindung C12-C15 Alkylbenzoat, welche besonders häufig als Lösungsmittel für UV-Lichtschutzfilter eingesetzt wird.

Zum Schutz gegen UV-B-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UV-A-Bereich, ist es wichtig, Filtersubstanzen zur Verfügung zu haben, da auch dessen Strahlen Schäden hervorrufen können. So ist erwiesen, dass UV-A-Strahlung zu einer Schädigung der elastischen und kollagenen Fasern des Bindegewebes führt, was die Haut vorzeitig altern lässt, und dass sie als Ursache zahlreicher phototoxischer und photoallergischer Reaktionen zu sehen ist. Der schädigende Einfluss der UV-B-Strahlung kann durch UV-A-Strahlung verstärkt werden.

Ein UV-B-Filter ist der 4,4',4''-(1,3,5-Triazin-2,4,6-triyltriimino)-tris-benzoesäure-tris(2-ethylhexylester), synonym: 2 , 4 , 6-Tris-[anilino(p-carbo-2'-ethyl-1'-hexyloxy)]- 1,3,5-triazin, **INCI**-Bezeichnung: Ethylhexyl Triazone. Diese UV-B-Filtersubstanz wird von der BASF AG unter der Warenbezeichnung UVINUL® T 150 vertrieben und zeichnet sich durch gute UV-Absorptionseigenschaften sowie gute Fotostabilität aus.

Der Hauptnachteil dieses UV-B-Filters ist die schlechte Löslichkeit in Lipiden. Gängige Lösungsmittel wie beispielsweise Isopropylpalmitat, Dicaprylylether, Isohexadecan, Butylenglycoldicaprylat/-dicaprat können maximal ca. 10 Gew.-% dieses Filters lösen, entsprechend etwa 1-1,5 Gew.-% gelöster, und damit aktiver, UV-Lichtschutzfiltersubstanz in der endgültigen Formulierung.

Die DE 25 19 433 beschreibt 2-Phenoxyethylester zur Verwendung als Bodenfungi-Inhibierungsmittel.

Die WO 00/68 176 A1 offenbart zum Beispiel Phenoxyethyl Laurate als Lösungsvermittler für UV-Lichtschutzfiltersubstanzen.

Weiterhin sind Phenoxyethylester beschrieben als potentielle Weichmacher für PVC in den Zeitschriften Plasticheskie Massy (1974), Band 5, Seiten 58-60 und Azerbaidzhanskii Khimicheskii Zhurnal (1968), Band 6, Seiten 122-123.

Phenoxyethylisobuttersäureester (Abkürzung: PEIB, CAS Nr. 103-60-6) wird als Duftstoff und Lebensmittelinhaltsstoff verwendet und hat die FEMA Nr. 2873. Die Verbindung hat von der US Food and Drug Administration (FDA) den Status GRAS (Generally Recognized as Safe) zuerkannt und damit die Unbedenklichkeit für den Lebensmittelbereich bekommen.

Die Herstellung von Phenoxyalkylestern erfolgt nach dem Fachmann bekannten Verfahren. Die Ester können beispielsweise durch Veresterung von Phenoxyalkylalkoholen mit C6-C12 Alkyl- und Cycloalkylcarbonsäuren hergestellt werden. Ebenso kann die Herstellung durch Umesterung von Phenoxyalkylalkoholen mit C6-C12 Alkyl- und Cycloalkylcarbonsäureestern, insbesondere Methyl-, Ethyl- oder Isopropylestern, erfolgen.

Beispiele für Phenoxyalkylalkohole sind 2-Phenoxyethanol und Phenoxypropanol. Kommerziell erhältliches Phenoxypropanol ist häufig eine Mischung aus 1-Phenoxy-2-propanol (CAS Nr. 770-35-4) und 2-Phenoxy-1-propanol (CAS Nr. 4169-04-4).

Beispiele für C6-C12 Alkyl- und Cycloalkylcarbonsäuren sind Capronsäure (Hexansäure), Cyclopentancarbonsäure, 2-Methylpentansäure, Heptansäure, Cyclohexancarbonsäure, Caprylsäure (Octansäure), 2-Ethylhexansäure, Sorbinsäure, Pelargonsäure (Nonansäure), Isononansäure, 3,5,5-Trimethylhexansäure, Caprinsäure, 2-Propylheptansäure, iso-Dekansäure, Undecansäure, Laurinsäure (Dodecansäure), 2-Butyloktansäure oder Mischungen dieser Carbonsäuren.

Die Umesterung kann ohne oder mit Katalysatoren bei Temperaturen zwischen 100 und 300 °C durchgeführt werden. Als Katalysatoren kommen insbesondere Lewis- oder Brönsted-Säuren in Betracht. Besonders bevorzugte Katalysatoren sind Sulfonsäuren, wie z.B. para-Toluolsulfonsäure, Zinn-Verbindungen, wie z.B. Zinnoxalat, Zinnoxid, Dibutylzinndiacetat, Dibutylzinnoxid oder Dibutylzinnlaurat, und Titan-Verbindungen, wie z.B. Tetrapropyltitanat, Tetraisopropyltitanat oder Tetrabutyltitanat.

Der Geruch der durch Veresterung oder Umesterung erhaltenen Produkte kann je nach Bedarf durch übliche Verfahren verbessert werden, indem leicht flüchtige Bestandteile entfernt werden, üblicherweise durch Destillation, Wasserdampf- oder Gasstrippen und andere Methoden der Desodorierung. Hierzu werden die Produkte bei 50 - 200 °C und 0 bis 1013 mbar behandelt. Weiterhin können Adsorptionsmittel, wie z.B. in EP 1585801 beschrieben, zur Geruchsverbesserung eingesetzt werden.

Ebenso kann die Farbe gegebenenfalls durch Behandlung mit Wasserstoffperoxid, Natriumchlorit, hypophosphoriger Säure und andere, dem Fachmann bekannte Methoden verbessert werden. Beispiele sind u.a. in der US 2005/0008586 beschrieben.

**Unter dem Begriff** " C 6-C12 Alkyl- und Cycloalkylcarbonsäuren" sind alle Carbonsäuren zusammengefasst, welche eine C-Zahl von größer-gleich 6 und kleiner-gleich 12 aufweisen. Umfasst sind sowohl aliphatische, gesättigte, einfach sowie mehrfach ungesättigte, lineare, verzweigte und cyclische Carbonsäuren. Beispiele sind: Capronsäure, Cyclopentancarbonsäure, 2-Methylpentansäure, Heptansäure, Cyclohexancarbonsäure, Caprylsäure, 2-Ethylhexansäure, Sorbinsäure, Isononansäure, 3, 5, 5-Trimethylhexansäure, Caprinsäure, 2-Propylheptansäure, iso-Dekansäure, Undecansäure, Undecylensäure, 2-Butyloktansäure.

Unter dem Begriff "Derivat" sind erfindungsgemäß strukturell eng verwandte Abkömmlinge einer chemischen Grundstruktur zu verstehen, die gleiche strukturelle Elemente aufweisen, aber verschiedene Substituenten tragen.

Aufgabe der Erfindung war es, einen Ölkörper bereitzustellen, der neben guten sensorischen Eigenschaften, wie Farbe, Geruch und Hautgefühl, auch eine gute Löslichkeit für aktive Substanzen, insbesondere für organische UV-Lichtschutzfilter, wie z.B. Triazine, so wie für Deodorant- und Antitranspirantwirkstoffe, besitzt.

Weitere nicht explizit genannte Aufgaben ergeben sich aus dem Kontext der nachfolgenden Beschreibung, der Beispiele sowie der Ansprüche.

### Beschreibung der Erfindung

Überraschenderweise wurde gefunden, dass Phenoxyalkylester von C6-C12 Alkyl- und Cycloalkylcarbonsäuren wie in Anspruch 1 beschrieben diese Anforderungen erfüllen. Ein Gegenstand der Erfindung ist die in Anspruch 1 beschriebene Verwendung von Phenoxyalkylestern von C6-C12 Alkyl- und Cycloalkylcarbonsäuren zur Herstellung eines kosmetischen, dermatologischen oder pharmazeutischen Sonnenschutzpräparats.

Ein weiterer Gegenstand der Erfindung sind kosmetische, dermatologische oder pharmazeutische Formulierungen enthaltend Phenoxyalkylester von C6-C12 Alkyl- und Cycloalkylcarbonsäuren wie in Anspruch 5 beschrieben.

Die erfindungsgemäße Verwendung von Phenoxyalkylestern der C6-C12 Alkyl- und Cycloalkylcarbonsäuren und die erfindungsgemäßen Phenoxyalkylester von C6-C12 Alkyl- und Cycloalkylcarbonsäuren enthaltenden Formulierungen, werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Beschreibung Dokumente zitiert, so soll deren Inhalt vollständig zum Offenbarungsgehalt der vorliegenden Erfindung gehören. Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen. Alle angegebenen Prozent (%) sind wenn nicht anders angegeben Massenprozent.

Als Phenoxyalkylester von C6-C12 Alkyl- und Cycloalkylcarbonsäuren wird im Folgenden mindestens eine Substanz der allgemeinen Formel (I) eingesetzt 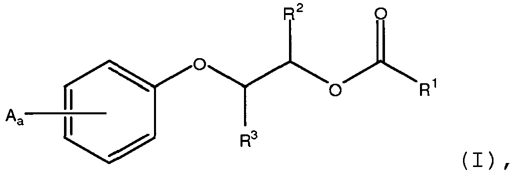 wobei
die Gruppe OOC-R1 der Säurerest der Säuren ausgewählt aus der Gruppe:
Cyclohexancarbonsäure, Caprylsäure, 2-Ethylhexansäure, Isononansäure, 3,5,5-Tri-methyl-hexansäure, 2-Propylheptansäure und 2 Butyloktansäure, so wie Mischungen enthaltend mindestens zwei dieser Carbonsäuren ist
R² und R³ unabhängig voneinander, gleich oder verschieden, Wasserstoff oder Methyl und
Aₐ mit a=1-5 unabhängig voneinander, gleich oder verschieden ausgewählt aus der Gruppe: H, F, Cl, Alkyl- oder Fluoralkylrest, CN, CO₂R⁴, OH, OR⁵, O₂CR⁶, NR⁷R⁸, NO₂, SO₃R⁹, mit R⁴ bis R⁹ unabhängig voneinander, gleich oder verschieden ausgewählt aus der Gruppe: H, Alkyl- oder Fluoralkylrest, sind.

Bevorzugt werden Substanzen der allgemeinen Formel (I) eingesetzt mit A unabhängig voneinander, gleich oder verschieden ausgewählt aus der Gruppe: H, F, Alkylrest, CN, OH, OR⁵, wobei als Alkylrest Methyl und Ethyl besonders bevorzugt sind.

Des Weiteren werden bevorzugt Substanzen der allgemeinen Formel (I) eingesetzt mit R⁴ bis R⁹ unabhängig voneinander, gleich oder verschieden H oder Alkylrest, wobei als Alkylrest Methyl, Ethyl, n-Propyl, i-Propyl, n-Butyl, s-Butyl, i-Butyl, t-Butyl bevorzugt und Methyl und Ethyl besonders bevorzugt sind.

Bevorzugt werden Substanzen der allgemeinen Formel (I) mit a=1 eingesetzt, besonders bevorzugt mit a = 1 und A = H.

Bevorzugte Kombinationen von R² und R³ sind ausgewählt aus der Gruppe
R² = R³ = H,
R² = H, R³ = Methyl,
R² = Methyl, R³ = H,
so wie Kombinationen dieser,
wobei R² = R³ = H besonders bevorzugt ist.

Bestimmt werden diese Reste durch die in der Synthese der Phenoxyalkylester eingesetzen Phenoxyalkylalkohole, wie beispielsweise 2-Phenoxyethanol und Phenoxypropanol. Kommerziell erhältliches Phenoxypropanol ist häufig eine Mischung aus 1-Phenoxy-2-propanol (CAS Nr. 770-35-4) und 2-Phenoxy-1-propanol (CAS Nr. 4169-04-4).

Besonders bevorzugte Verbindungen der allgemeinen Formel (I) sind: 2-Phenoxyethyl-oktansäureester, 2-Phenoxyethyl-2-ethylhexansäureester, 2-Phenoxyethylcyclohexancarbonsäureester, 2-Phenoxyethylisononansäureester, 2-Phenoxyethyl-3,5,5-trimethylhexansäureester, 2-Phenoxyethyl-2-butyl-oktansäureester, 1-Phenoxy-2-propyloktansäureester, 1-Phenoxy-2-propyl-2-ethylhexansäureester, 1-Phenoxy-2-propylcyclohexancarbonsäureester, 1-Phenoxy-2-propylisononansäureester, 1-Phenoxy-2-propyl-3,5,5-trimethylhexansäureester oder 1-Phenoxy-2-propyl-2-butyl-oktansäureester.

Erfindungsgemäß wird mindestens eine Substanz der allgemeinen Formel (I) zur Herstellung von Sonnenschutzpräparaten verwendet.

Bevorzugt können auch Mischungen der Phenoxyalkylester verwendet werden.

Die kosmetischen, dermatologischen oder pharmazeutischen Formulierungen können z. B. mindestens eine zusätzliche Komponenten enthalten, ausgewählt aus der Gruppe der
Emollients,
Emulgatoren und Tenside,
Verdicker/Viskositätsregler/Stabilisatoren,
UV-Lichtschutzfilter,
Antioxidantien und Vitamine,
Hydrotrope (oder Polyole),
Fest- und Füllstoffe,
Filmbildner,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Biogene Wirkstoffe,
Pflegeadditive,
Überfettungsmittel,
Lösungsmittel.

Als Emollients können alle kosmetischen Öle insbesondere Mono- oder Diester von linearen und/oder verzweigten Mono-und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z.B. im Jojobaöl oder im Spermöl vorliegen. Geeignete Dicarbonsäureester sind z.B. Di-n-butyl-adipat, Di-n-butyl-sebacat, Di-(2-ethylhexyl)-adipat, Di-(2-hexyldecyl)-succinat, D-isotridecylacelaat. Geeignete Diolester sind z.B. Ethylenglycoldioleat, Ethylenglycol-di-isotridecanoat, Propylenglycol-di-(2-ethylhexanoat), Butandiol-di-isostearat, Butandiol-di-caprylat/caprat und Neopentylglycol-di-caprylat. Weitere Fettsäureester, die als Emollients eingesetzt werden können, sind z.B. C₁₂-₁₅ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Haifischlebertran, Dorschleberöl, Walöl, Rindertalg und Butterfett, Wachse wie Bienenwachs, Karnaubapalmwachs, Spermazet, Lanolin und Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxy- substituierte Polymethylsiloxane oder Cyclomethylsiloxane. Als weitere Ölkörper kommen beispielsweise Guerbetalkohole auf Basis von Fettalkoholen mit 6 bis 18, vorzugsweise 8 bis 10 Kohlenstoffatomen, Ester von linearen C6-C22-Fettsäuren mit linearen C6- C22-Fettalkoholen, Ester von verzweigten C6-C13-Carbonsäuren mit linearen C6-C22-Fettalkoholen, Ester von linearen C6-C22-Fettsäuren mit verzweigten C8-C18-Alkoholen, insbesondere 2-Ethylhexanol oder Isononanol, Ester von verzweigten C6-C13-Carbonsäuren mit verzweigten Alkoholen, insbesondere 2-Ethylhexanol oder Isononanol, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C6-C10-Fettsäuren, flüssige Mono-/Di-/Triglyceridmischungen auf Basis von C6-C18-Fettsäuren, Ester von C6-C22-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C6-C22-Fettalkoholcarbonate, Guerbetcarbonate, Ester der Benzoesäure mit linearen und/oder verzweigten C6-C22-Alkoholen (z. B. Finsolv^{™} TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als Emulgatoren oder Tenside können nichtionische, anionische, kationische oder amphotere Tenside eingesetzt werden.

Als nichtionogene Emulgatoren oder Tenside können Verbindungen aus mindestens einer der folgenden Gruppen eingesetzt werden:
Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
C_{12/18}-Fettsäuremono- und -diester von Anlagerungsprodukten von 1 bis 100 Mol Ethylenoxid an Glycerin,
Glycerinmono- und -diester und Sorbitanmono- und -diester von gesättigten und ungesättigten Fettsäuren mit 6 bis 22 Kohlenstoffatomen und deren Ethylenoxidanlagerungsprodukte, Alkylmono- und -oligoglycoside mit 8 bis 22 Kohlenstoffatomen im Alkylrest und deren Ethylenoxidanlagerungsprodukte,
Anlagerungsprodukte von 2 bis 200 Mol Ethylenoxid an Ricinusöl und/oder gehärtetes Ricinusöl,
Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate sowie Mono-, Di- und/oder Tri-PEG-alkylphosphate und deren Salze,
Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15,
Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL^{®} EM 90 (Evonik Goldschmidt GmbH)),
Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, wie z.B. Glycerin oder Polyglycerin,
Zitronensäureester wie z.B. Glyceryl Stearate Citrate, Glyceryl Oleate Citrate und Dilauryl Citrate.

Anionische Emulgatoren oder Tenside können wasserlöslich machende anionische Gruppen wie z.B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.

Auch kationische Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid.

Weiterhin können Monoalkylamidoquats wie z.B. Palmitamidopropyltrimethylammoniumchlorid oder entsprechende Dialkylamidoquats eingesetzt werden.

Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Emulgatoren beigesetzt sein.

Typische Beispiele für milde, d. h. besonders **hautverträgliche Tenside sind** Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere beispielsweise auf Basis von Weizenproteinen.

Weiterhin ist es möglich, amphotere Tenside wie z.B. Betaine, Amphoacetate oder Amphopropionate einzusetzen, so **z.B. Substanzen** wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.

Von den ampholytischen Tensiden können solche oberflächenaktiven Verbindungen eingesetzt werden, die außer einer C8/18-Alkyl- oder -Acylgruppe im Molekül mindestens eine freie Aminogruppe und mindestens eine - COOH- oder -SO₃H- Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-**Alkyliminodipropionsäuren,** N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Weitere Beispiele ampholytischer Tenside sind das N-**Kokosalkylaminopropionat, das** Kokosacylaminoethylaminopropionat und das C12/18-Acylsarcosin.

Geeignete Verdicker sind beispielsweise Polysaccharide, insbesondere Xanthan-Gum, Guar-Guar, Agar-Agar, Alginate u n d Tylosen, Carboxymethylcellulose und Hydroxyethylcellulose, ferner höhermolekulare Polyethylenglycolmono- und -di-ester von Fettsäuren, Polyacrylate, (z. B. Carbopole TM oder Synthalene TM ), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und /oder Metallsalze von Fettsäuren, wie z.B. Magnesium-, Aluminium- und/oder Zinkstearat bzw. -ricinoleat eingesetzt werden.

Als Viskositätsregler für wässrige Tensidsysteme können z.B. NaCl, niedermolekulare nichtionische Tenside, wie Cocoamide DEA/MEA und Laureth-3, **oder** polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen:
3-Benzylidencampher und dessen Derivate, z.B. 3-(4-Methylbenzyliden)campher,
4-**Aminobenzoesäurederivate, wie z.B.** 4-(Dimethylamino)benzoesäure-2-ethylhexylester,4-(Dimethylamino)benzoesäure-2-ethylhexylester und 4-(Dimethylamino)benzoesäureamylester
Ester der Zimtsäure, wie z.B. 4-Methoxyzimtsäure-2-ethylhexylester, 4-Methoxyzimtsäureisopentylester, 2-Cyan-3-phenyl-zimtsäure-2-ethylhexylester (Octocrylene),
Ester der Salicylsäure, wie z.B. Salicylsäure-2-ethylhexylester, **Slicylsäure**-4-isopropylbenzylester, Salicylsäurehomomenthylester,
**Derivate des Benzophenons, wie z.B.** 2-Hydroxy-4-methoxybenzophenon, 2-Hydroxy-4-methoxy-4'-methylbenzophenon, 2,2'-Dihydroxy-4-methoxybenzophenon,
**Ester der Benzalmalonsäure, wie z.B.** 4-Methoxybenzmalonsäuredi-2-ethylhexyester,
Triazinderivate, wie z.B. 2,4,6-Trianilino-(p-carbo-2'-ethyl-1'-hexyloxy)-1,3,5-triazin, Octyltriazon und solche in der EP 1180359 und DE 2004/027475 beschriebenen,
Propan-1,3-dione, wie z.B. 1-(4-tert.Butylphenyl)-3-(4'-methoxyphenyl)propan-1,3-dion.

Als wasserlösliche UVB-Lichtschutzfilter kommen in Frage:
2-Phenylbenzimidazol-5-sulfonsäure und deren Alkali-, Erdalkali-, Ammonium-, Alkylammonium-, Alkanolammonium- und Glucammoniumsalze,
Sulfonsäurederivate von Benzophenon, wie z.B. 2-Hydroxy-4-methoxybenzophenon-5-sulfonsäure und ihre Salze,
Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.

Weitere geeignete UV-Lichtschutzfilter sind der Übersicht von P. Finkel in SÖFW-Journal 122, 543 (1996) zu entnehmen.

Neben den beiden vorgenannten Gruppen primärer **UV-Lichtschutzfilter können auch sekundäre** Lichtschutzmittel vom Typ der Antioxidantien eingesetzt werden, die die photochemische Reaktionskette unterbrechen, welche ausgelöst wird, wenn UV-Strahlung in die Haut eindringt.

Als Antioxidantien und Vitamine können z.B. Superoxid-Dismutase, Tocopherol (Vitamin E), Tocopherolsorbat, Tocopherolacetat, **andere Ester** von Tocopherol, Dibutylhydroxytoluol und Ascorbinsäure (Vitamin C) und ihre **Salze sowie deren Derivate** (z.B. Magnesium Ascorbylphosphat, Natrium Ascorbylphosphat, Ascorbylsorbat), Ascorbyl Ester von Fettsäuren, butylierte Hydroxybenzoesäure und ihre Salze, Peroxide wie z.B. Wasserstoffperoxid, Perborate, Thioglycolate, Persulfatsalze, 6-Hydroxy-2,5,7,8-Tetramethylchroman-2-Carboxylsäure (TROLOX.RTM), Gallussäure und ihre Alkylester, Harnsäure und ihre Salze und Alkylester, Sorbinsäure und ihre Salze, Liponsäure, Ferulasäure, Amine (z.B. N,N-Diethylhydroxylamin, Amino-Guanidine), Sulfhydryl-Verbindungen (z.B. Glutathion), Dihydroxy-Fumarsäure und ihre Salze, Glycinpidolat, Argininpidolat, Nordihydroguaiaretissche Säure, Bioflavonoide, Curcumin, Lysin, L-Methionin, Prolin, Superoxid Dismutase, Silymarin, Tee Extract, Grapefruit Schalen/Kern Extrakt, Melanin, Rosmarin Extrakt, Thioctansäure, Resveratrol, Oxyresveratrol, etc. eingesetzt werden.

Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele sind:
Glycerin Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton, technische Oligoglyceringemische mit einem Eigenkondensationsgrad von 1,5 bis 10 wie etwa technische Diglyceringemische mit einem Diglyceringehalt von 40 bis 50 Gew.-%,
Methylolverbindungen, wie insbesondere Trimethylolethan, Trimethylolpropan, Trimethylolbutan, Pentaerythrit und Dipentaerythrit, Niedrigalkylgucoside, insbesondere solche mit 1 bis 4 Kohlenstoffatomen im Alkylrest, wie beispielsweise Methyl- und Butylglucosid, Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose, Aminozucker, wie beispielsweise Glucamin.

Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, **Polymerpartikel wie etwa Polyacrylat-** oder **Polymethylacrylatpartikel oder Siliconelastomere.** Einsetzbare Füllstoffe umfassen Stärke und Stärkederivate, wie Tapiocastärke, Distärkephosphat, Aluminium- bzw. Natrium-Stärke, Octenylsuccinat sowie Pigmente, die weder hauptsächlich UV-Filter- noch färbende Wirkung haben, beispielsweise Aerosile® (CAS-Nr. 7631-86-9).

Als Filmbildner zur z.B. Verbesserung der Wasserfestigkeit können beispielsweise eingesetzt werden: Polyurethane, Dimethicone, Copolyol, Polyacrylate oder PVP/VA Copolymer (PVP = Polyvinylpyrrolidon, VA = Vinylacetat). Als fettlösliche Filmbildner können eingesetzt werden: z.B. Polymere auf Basis von Polyvinylpyrrolidon (PVP), Copolymere des Polyvinylpyrrolidons, PVP/Hexadecen-Copolymer oder das PVP/Eicosen-Copolymer.

Als Perlglanzadditive können z.B. Glycoldistearate oder PEG-3 Distearat eingesetzt werden.

Als Deodorantwirkstoffe kommen z.B. Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Illit, Beidelit, Nontronit, Saponit, Hectorit, Bentonit, Smectit, ferner beispielsweise Zinksalze der Ricinolsäure in Frage. Keimhemmende Mittel sind ebenfalls geeignet, eingearbeitet zu werden. Keimhemmende Substanzen sind zum Beispiel 2,4,4'-Trichlor-2'-hydroxydiphenylether (Irgasan), 1,6-Di-(4-chlorphenylbiguanido)-hexan (**Chlorhexidin**), 3,4,4'-Trichlorcarbonilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO® Cosmo P813, Evonik Goldschmidt GmbH), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 666 732 beschriebenen wirksamen Agenzien.

Als Antitranspirantwirkstoffe können Adstringentien eingesetzt werden, beispielsweise basische Aluminiumchloride wie Aluminiumchlorhydrat ("ACH") und Aluminium-Zirkonium-Glycine-Salze ("ZAG").

Als Insekten-Repellentien können beispielsweise N,N-Diethyl-m-toluamid, 1,2-Pentandiol oder Insect Repellent 3535 eingesetzt werden.

Als Selbstbräuner können z.B. Dihydroxyaceton und Erythrulose eingesetzt werden.

Als Konservierungsstoffe können beispielsweise Mischungen einzelner oder mehrerer Alkylparabenester mit Phenoxyethanol eingesetzt werden. Bei den Alkylparabenestern kann es sich um Methylparaben, Ethylparaben, Propylparaben und/oder Butylparaben handeln. Anstelle von Phenoxyethanol können auch andere Alkohole eingesetzt werden, wie beispielsweise Benzylalkohol oder Ethanol. Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate, Methylisothiazolin, Chlormethylisothiazolin, Ethylhexylglycerin oder Caprylyl Glycol eingesetzt werden.

Als Konditioniermittel können z.B. organische quaternäre **Verbindungen wie Cetrimoniumchlorid,** Dicetyldimoniumchlorid, Behentrimoniumchlorid, Distearyldimoniumchlorid, Behentrimoniummethosulfat, Distearoylethyldimoniumchlorid, **Palmitamidopropyltrimoniumchlorid, Guar** Hydroxypropyltrimoniumchlorid, Hydroxypropylguar Hydroxypropyltrimoniumchlorid, oder Quaternium-80 oder auch Aminderivate wie z.B. Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Als Farbstoffe können die für kosmetische Zwecke geeigneten und zugelassenen Substanzen eingesetzt werden, wie sie beispielsweise in der Publikation "Kosmetische Färbemittel" d e r Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Polyphenole, Desoxyribonucleinsäure, Coenzym Q10, Retinol, AHA-Säuren, Aminosäuren, Hyaluronsäure, alpha-Hydroxysäuren, Isoflavone, Polyglutaminsäure, Creatin (und Creatinderivate), Guanidin (und Guanidinderivate), Pseudoceramide, essentielle Öle, Peptide, Proteinhydrolysate, Pflanzenextrakte, Bisabolol, Allantoin, Panthenol, Phytantriol, Idebenon, Lakritz Extrakt, Glycyrrhizidin und Idebenon, Skleroglucan, β-Glucan, Santalbinsäure und Vitaminkomplexe zu verstehen. Beispiele für Pflanzenextrakte sind Kastanien Extrakt, Kamillen Extrakt, Rosmarin Extrakt, schwarze und rote Johannisbeer Extrakt, Birken Extrakt, Hagebutten Extrakt, Algen Extrakte, Grüner Tee Extrakt, Aloe Extrakt, Ginseng Extrakt, Ginko Extrakt, Grapefruit Extrakt, Calendula Extrakt, Kampher, Thymus Extrakt, Mangosteen Extrakt, Cystus Extrakt, Terminalia Arjuna Extrakt, Hafer Extrakt, Oregano Extrakt, Himbeer Extrakt, Erdbeer Extrakt, etc.

Zu den biogenen Wirkstoffen können auch die sogenannten Barriere Lipids gezählt werden, für welche beispielhaft Ceramide, Phytosphingosin und Derivate, Sphingosin und Derivate, Sphinganin und Derivate, Pseudoceramide, Phospholipide, Lysophospholipide, Cholesterin und Derivate, Cholesteryl Ester, freie Fettsäuren, Lanolin und Derivate, Squalan, Squalen und verwandte Substanzen genannt werden. Zu den biogenen Wirkstoffen werden im Sinne der Erfindung auch anti-Akne wie z.B. Benzylperoxid, Phytosphingosin und Derivate, Niacinamid Hydroxybenzoat, Nicotinaldehyd, Retinolsäure und Derivate, Salicylsäure und Derivate, Citronellsäure etc. und anti-Cellulite wie z.B. Xanthin Verbindungen wie Coffein, Theophyllin, Theobromin und Aminophyllin, Carnitin, Carnosin, Salicyloyl Phytosphingosin, Phytosphingosine, Santalbinsäure etc. gezählt, ebenso wie Antischuppenmittel wie beispielsweise Salicylsäure und Derivate, Zink Pyrithion, Selensulfid, Schwefel, Ciclopiroxolamin, Bifonazol, Climbazol, Octopirox und Actirox etc, ebenso wie Adstringetien wie z.B. Alkohol, Aluminium Derivate, Gallsäure, Pyridoxinsalicylat, Zinksalze wie z.B. Zinksulfat, -acetat, -chlorid, -lactat, Zirconiumchlorohydrate etc. Ebenso können Bleichmittel wie Kojisäure, Arbutin, Vitamin C und Derivate, Hydroquinon, Turmeric oil, Creatinin, Sphingolipide, Niacinamid, etc. zu den biogenen Wirkstoffen gezählt werden.

Als Pflegeadditive können z.B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Lösungsmittel können z.B. aliphatische Alkohole wie Ethanol, Propanol oder 1,3-Propandiol, cyclische Carbonate wie Ethylencarbonat, Propylencarbonat, Glycerincarbonat, Ester von Mono- oder Polycarbonsäuren wie Ethylacetat, Ethyllactat, Dimethyladipat und Diethyladipat, Propylenglycol, Dipropylenglycol, Glycerin, Glycerincarbonat oder Wasser eingesetzt werden.

Erfindungsgemäße Verwendung eignet sich für die Herstellung von Sonnenschutzpräparaten.

Ein weiterer Gegenstand der Erfindung ist daher die Verwendung von Phenoxyalkylester von C6-C12 Alkyl- und Cycloalkylcarbonsäuren der allgemeinen Formel (I) in kosmetischen, dermatologischen oder pharmazeutischen Formulierungen als Lösungsvermittler oder Lösungsmittel von mindestens einer der nachfolgend genannten UV-Lichtschutzfiltersubstanzen.

Bei der Verwendung der Phenoxyalkylester von C6-C12 Alkyl- und Cycloalkylcarbonsäuren der allgemeinen Formel (I) als Lösungsvermittler oder Lösungsmittel von UV-Lichtschutzfiltersubstanzen werden jene Phenoxyalkylester eingesetzt, die bei der Verwendung der oben beschriebenen Gruppen von Phenoxyalkylestern zur Herstellung der Formulierungen beschrieben wurden. Es werden die lipophilen, hydrophoben UV-Lichtschutzfiltersubstanzen der Triazinderivate
oder die UV-Lichtschutzfiltersubstanzen ausgewählt aus 2-Cyano-3-phenyl-zimtsäure-2-ethylhexylester, 2,4-Bis-{[4-(2-ethyl-hexyloxy)-2-hydroxy]-phenyl}-6-(4-methoxyphenyl)-1,3,5-triazin, Dioctylbutylamidotriazon, 2-Hydroxy-4-methoxybenzophenon, 2-Hydroxy-4-methoxy-4'-methyl-benzophenon, 2,2'-Dihydroxy-4-methoxybenzophenon, 4-Methoxybenzmalonsäuredi-2-ethylhexylester, 2,4,6-Tris-[anilino-(p-carbo-2'-ethyl-1'-hexyloxy)]-1,3,5-triazin, 2,4-bis-[5,1(dimethylpropyl)benzoxazol-2-yl-(4-phenyl)-imino]-6-(2-ethylhexyl)-imino-1,3,5-triazin, 2,4-Bis-{[4-(2-Ethyl-hexyloxy)-2-hydroxy]-phenyl}-6-(4-methoxyphenyl)-1,3,5-triazin und 2-[4,6-Bis(2,4-dimethylphenyl)-1,3,5-triazin-2-yl]-5-(octyloxy)phenol eingesetzt.

Als UV-A-Filter werden vorzugsweise 1-(4'-tert.-Butylphenyl)-3-(4'-methoxyphenyl)propan-1,3-dion, 1-Phenyl-3-(4'-isopropylphenyl)-propan-1,3-dion eingesetzt.

Besonders bevorzugte UV-A-Filter sind 4-(tert.-Butyl)-4'-methoxydibenzoylmethan (CAS Nr. 70356-09-1), welches von Givaudan unter der Marke Parsol® 1789 und von Merck unter der Handelsbezeichnung Eusolex® 9020 verkauft wird, und Hydroxybenzophenone gemäß DE 102004027475, besonders bevorzugt der 2-(4'-Diethylamino-2'-hydroxybenzoyl)-benzoesäure-hexylester (auch: Aminobenzophenon), welcher unter Namen Uvinul A Plus bei der Fa. BASF erhältlich ist.

Weiterhin bevorzugte UV-Filtersubstanzen sind ferner sogenannte Breitbandfilter, d.h. Filtersubstanzen, die sowohl UV-A- als auch UV-B-Strahlung absorbieren. Innerhalb dieser Gruppe werden bevorzugt 2,2'-Methylen-bis-(6-(2H-benzotriazol-2-yl)-4-(1,1,3,3-tetramethylbutyl)-phenol, welches unter der Handelsbezeichnung Tinosorb® M bei der CIBA-Chemikalien GmbH erhältlich ist und 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, eingesetzt.

Die Einsatzmenge der UV-Lichtschutzfilter beträgt vorzugsweise 0,01-15 %, bevorzugt 0,05-10 %, besonders bevorzugt 0,1-5 % bezogen auf die Formulierung.

Bevorzugt ist der Einsatz einer Kombination aus mehreren verschiedenen UV-Filtern.

Eine weitere, bevorzugt eingesetzte, zusätzliche Komponente ist die Gruppe der Filmbildner, um die Wasserfestigkeit der Zusammensetzungen zu verbessern und somit auch die UV-Schutzleistung zu erhöhen. Bevorzugt eingesetzte Filmbildner sind Polyurethane, Dimethicone, Copolyol, Polyacrylate, PVP/VA Copolymer (PVP = Polyvinylpyrrolidon, VA = Vinylacetat), Polyvinylpyrrolidon (PVP), Copolymere des Polyvinylpyrrolidons, PVP/Hexadecen-Copolymer oder das PVP/Eicosen-Copolymer.

Gegenstand der Erfindung sind außerdem kosmetische, dermatologische oder pharmazeutische Formulierungen, enthaltend eine Substanz der allgemeinen Formel (I)

Die erfindungsgemäßen, kosmetischen, dermatologischen oder pharmazeutische Formulierungen werden erhalten durch die Verwendung der oben beschriebenen Gruppen von Phenoxyalkylestern zur Herstellung der Formulierungen wie auch zur Verwendung als Lösungsvermittler. Ebenso enthalten die kosmetischen, dermatologischen oder pharmazeutische Formulierungen bevorzugt weitere zusätzliche Komponenten, als dass diese zusätzlichen Komponenten bevorzugt wie oben beschrieben bei der Verwendung als Lösungsvermittler eingesetzt werden.

Erfindungsgemäße Formulierungen können beispielsweise Verwendung als ein Hautpflege-, Gesichtspflege-, Kopfpflege-, Körperpflege, Intimpflege-, Fußpflege-, Haarpflege-, Nagelpflege, Zahnpflege- oder Mundpflegeprodukt finden.

Erfindungsgemäße Formulierungen können beispielsweise Verwendung in Form einer Emulsion, einer Suspension, einer Lösung, einer Creme, einer Salbe, einer Paste, eines Gels, eines Öls, eines Puders, eines Aerosols, eines Stiftes, eines Sprays, eines Schaums, eines Reinigungsproduktes, eines Schmink- oder Sonnenschutzpräparates oder eines Gesichtswassers finden.

Die kosmetischen, dermatologischen oder pharmazeutischen Formulierungen enthalten 3 bis 60 Gewichtsprozent, bevorzugt 3 bis 25 Gewichtsprozent und besonders bevorzugt 3 bis 15 Gewichtsprozent mindestens einer Substanz der allgemeinen Formel (I).

Die kosmetischen, dermatologischen oder pharmazeutischen Formulierungen enthalten:
(a) 3 - 60 Gewichtsprozent mindestens einer Substanz der allgemeinen Formel (I),
(b) 0,1 - 20 Gewichtsprozent Tenside und/oder Emulgatoren und/oder Coemulgatoren,
(c) 0,1 - 40 Gewichtsprozent weiterer Ölkörper und
(d) 0 - 98 Gewichtsprozent Wasser,
wobei die Gewichtsprozent der Komponenten (a), (b), (c) und (d) sich auf 100 Gewichtsprozent addieren.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Ausführungsbeispiele:

### Beispiel 1: Herstellung 2-Phenoxyethyl-oktansäureester

Es wurden 96,8 g (0,70 mol) 2-Phenoxyethanol und 112,2 g (0,78 mol) Caprylsäure zusammen mit 1,1 g para-Toluolsulfonsäure 6 h bei 150-160 °C am Wasserabscheider erhitzt. Die überschüssige Säure wurde im Vakuum abdestilliert und das Reaktionsgemisch abgekühlt. Der Katalysator wurde mit Kaliumhydroxidlösung neutralisiert und das Produkt anschließend mit Wasserstoffperoxid gebleicht und desodoriert. Man erhält nach Filtration 176,5 g eines geruchlosen Öls: OHZ < 1; SZ = 0,1; Farbzahl (APHA) = 52 Hazen.

### Beispiel 2: Herstellung 2-Phenoxyethyl-oktansäureester

Es wurden 193,7 g (1,40 mol) 2-Phenoxyethanol und 206,3 g (1,43 mol) Caprylsäure zusammen mit 0,4 g TEGOKAT® 188 (Zinn(II)-oxid; 0,1 Gew.-% **bezogen auf die** Gesamtzusammensetzung) 5 h auf 220 °C am Wasserabscheider erhitzt. Die überschüssige Säure wurde im Vakuum abdestilliert und das Reaktionsgemisch abgekühlt. Das Produkt wird mit Kaliumhydroxidlösung neutralisiert und desodoriert. Nach Zugabe von 0,8 g TEGO® TINEX P wurde das Produkt filtriert und man erhält 362,8 g eines geruchlosen Öls: OHZ = 1,2; SZ = 0,1; Farbzahl (APHA) = 56 Hazen.

### Beispiel 3: Herstellung 2-Phenoxyethyl-2-ethylhexansäure-ester

Es wurden 96,8 g (0,70 mol) 2-Phenoxyethanol und 103,2 g (0,72 mol) 2-Ethylhexansäure zusammen mit 1,0 g para-Toluolsulfonsäure 8 h bei 150-160 °C am Wasserabscheider erhitzt. Das Reaktionsgemisch wurde abgekühlt und der Katalysator mit Kaliumhydroxidlösung neutralisiert. Anschließend wurde das Produkt mit Wasserstoffperoxid gebleicht und desodoriert. Man erhält nach Filtration 175,0 g eines geruchlosen Öls: OHZ < 1; SZ = 0,2; Farbzahl (APHA) = 40 Hazen.

### Beispiel 4: Herstellung 2-Phenoxyethyl-cyclohexancarbonsäureester

Es wurden 111,6 g (0,81 mol) 2-Phenoxyethanol und 104,5 g (0,81 mol) Cyclohexancarbonsäure zusammen mit 1,0 g para-Toluolsulfonsäure 6 h bei 150-160 °C am Wasserabscheider erhitzt. Das Reaktionsgemisch wurde abgekühlt und mit Kaliumhydroxidlösung neutralisiert. Anschließend wurde das Produkt desodoriert. Man erhält nach Filtration 192,5 g eines geruchlosen Öls: OHZ < 1; SZ = 0,2; Farbzahl (APHA) = 70 Hazen.

### Beispiel 5: Herstellung 2-Phenoxyethyl-2-butyloktansäure-ester

Es werden 81,7 g (0,59 mol) 2-Phenoxyethanol und 118,3 g (0,59 mol) 2-Butyloktansäure (ISOCARB® 12, Fa. Sasol) zusammen mit 0,9 g para-Toluolsulfonsäure 6 h bei 150-160 °C am Wasserabscheider erhitzt. Das Reaktionsgemisch wird abgekühlt und mit Kaliumhydroxidlösung neutralisiert.

Anschließend wird das Produkt mit Wasserstoffperoxid gebleicht und desodoriert. Man erhält nach Filtration 185,3 g eines geruchlosen Öls: OHZ < 1; SZ = 0,2; Farbzahl (APHA) = 74 Hazen.

### Beispiel 6: Herstellung 1-Phenoxy-2-propyl-oktansäureester

Es wurden 106,3 g (0,70 mol) Phenoxypropanol (Fa. Bayer) und 103,7 g (0,72 mol) Caprylsäure zusammen mit 1,1 g para-Toluolsulfonsäure 6 h bei 150-160 °C am Wasserabscheider erhitzt. Das Reaktionsgemisch wurde abgekühlt und mit Kaliumhydroxidlösung neutralisiert. Anschließend wurde das Produkt mit Wasserstoffperoxid gebleicht und desodoriert. Man erhält nach Filtration 191,0 g eines geruchlosen Öls: OHZ < 1; SZ = 0,1; Farbzahl (APHA) = 85 Hazen.

### Beispiel 7: Herstellung 1-Phenoxy-2-propyl-cyclohexan-carbonsäureester

Es wurden 106,3 g (0,70 mol) Phenoxypropanol (Fa. Bayer) und 89,7 g (0,70 mol) Cyclohexancarbonsäure zusammen mit 1,0 g para-Toluolsulfonsäure 8 h bei 150-160 °C am Wasserabscheider erhitzt. Das Reaktionsgemisch wurde abgekühlt und mit Kaliumhydroxidlösung neutralisiert. Anschließend wird das Produkt mit Wasserstoffperoxid gebleicht und desodoriert. Man erhält nach Filtration 179,9 g eines geruchlosen Öls: OHZ = 1,1; SZ = 0,1; Farbzahl (APHA) = 62 Hazen.

### Beispiel 8: Lösungsvermögen von UV-Lichtschutzfiltern

Zum Test des Lösungsvermögens von kristallinen UV-Lichtschutzfiltern in Phenoxyalkylestern wurden stellvertretend vier repräsentative UV-A- bzw. UV-B-Lichtschutzfilter ausgewählt. Hierbei handelt es sich um Benzophenon-3 (2-Hydroxy-4-methoxy-benzophenon, BP-3), Butyl Methoxydibenzoylmethane (BMDM), Octyl Triazon (2,4,6-Trianilino-p-(carbo-2'ethylhexyl-1'-oxi)-1,3,5-triazine, EHT) und Bemotrizinol (bis-ethylhexyloxyphenol methoxyphenyl triazine, BEMT).

Das Lösungsvermögen von herkömmlichen Esterölen für diese Verbindungen ist in den meisten Fällen nicht befriedigend. Eine Verbindung mit gutem Lösungsvermögen für UV-Lichtschutzfiltersubstanzen ist das schon erwähnte Tegosoft^{®} TN, welches deshalb als Inhaltsstoff für Sonnenschutzformulierungen auch weithin etabliert ist.

Zur Bestimmung des Lösungsvermögens für diese drei UV-Lichtschutzfiltern wurde jeweils eine bestimmte Menge (50g) einer der erfindungsgemäßen Verbindungen vorgelegt und auf 22°C temperiert. Es wurde 1 Gewichtsprozent eines UV-Lichtschutzfilters zugegeben und gerührt, bis sich diese Menge vollständig und homogen gelöst hat. Dieser Vorgang wurde wiederholt, bis die maximal lösbare Menge des UV-Lichtschutzfilters überschritten wurde. Zum vollständigen Auflösen ist bei höheren Konzentrationen oftmals eine längere Rührzeit von mehreren Stunden erforderlich.

War auf diese Weise die Maximalkonzentration grob bestimmt worden, wurde zur Feinbestimmung der Konzentrationsbereich um diese Maximalkonzentration herum mit kleineren Einwaagemengen des UV-Lichtschutzfilters wiederholt.

Als Referenz wurde die Verbindung Tegosoft^{®} TN (C12-15 Alkyl Benzoate) verwendet.

| **Substanzname** | **Benzophenone-3** | **Tinosorb S** | **BMDM** | **Uvinul T 150** |
|---|---|---|---|---|
| Tegosoft® TN [als Vergleich] (INCI: C12-15 Alkyl Benzoate) | 15% | 9% | 13% | 4% |
| 2-Phenoxyethyl-hexansäureester* | 28% | 11% | 18% | 6% |
| 2-Phenoxyethyl-cyclohexancarbonsäureeste | 30% | 12% | 19% | 6% |
| 2-Phenoxyethyl-oktansäureester | 28% | 11% | 18% | 6% |
| 2-Phenoxyethyl-2-ethylhexansäureester | 25% | 12% | 17% | 4% |
| 2-Phenoxyethyl-isononansäureester | 25% | 9% | 16% | 4% |
| 2-Phenoxyethyl-2-propylheptansäureester | 20% | 9% | 13% | 4% |
| 2-Phenoxyethyl-2-butyloktansäureester | 19% | 8% | 14% | 5% |
| 1-Phenoxy-2-propyl-cyclohexancarbonsäureester | 27% | 10% | 16% | 6% |
| 1-Phenoxy-2-propyl-oktansäureester | 21% | 9% | 14% | 6% |
| 1-Phenoxy-2-propyl-2-ethylhexansäureester | 21% | 9% | 15% | 4% |

| | | | | |
|---|---|---|---|---|
| * nicht erfindungsgemäß | | | | |

Wie aus den obigen Werten ersichtlich, ist das Lösungsvermögen der erfindungsgemäßen Verbindungen deutlich besser als das Lösungsvermögen von Tegosoft^{®} TN.

### Beispiel 9: Kosmetische Formulierungen

Die erfindungsgemäßen Verbindungen der allgemeinen Formel (I) können sowohl in Öl-in-Wasser als auch in Wasser-in-Öl Emulsionen eingesetzt werden.

Die Formulierungsbeispiele sollen dazu dienen, die Verwendbarkeit von z.B. 2-Phenoxyethyl-oktansäureester in kosmetischen Emulsionen beispielhaft zu illustrieren und beschränken den Gegenstand der Erfindung nicht.

Alle Mengenangaben in % sind, soweit nicht anders vermerkt, Gewichtsteile. Herstellung und Homogenisierschritte erfolgen nach üblichen Methoden.
**Formulierungsbeispiel A:**

**O/W Sonnenschutzlotion**

| **Zusammensetzung** | **%w/w** |
|---|---|
| Polyglyceryl-3 Methylglucose Distearate (TEGO^{®} Care 450, Evonik Goldschmidt GmbH) | 3,0 |
| Glyceryl Stearate (TEGIN^{®} M Pellets, Evonik Goldschmidt GmbH) | 1,0 |
| **2-Phenoxyethyl-oktansäureester** | **8,5** |
| Simmondsia Chinensis (Jojoba) Seed Oil (Jojobaöl) | 1,5 |
| Ethylhexyl Salicylate | 5,0 |
| Bis-Ethylhexyloxyphenol Methoxyphenyl Triazine | 9,0 |
| Ethylhexyl Methoxycinnamate | 9,0 |
| Butyl Methoxydibenzoylmethane | 3,0 |
| Carbomer (TEGO^{®} Carbomer 134, Evonik Goldschmidt GmbH) | 0,2 |
| C12-15 Alkyl Benzoate (TEGOSOFT® TN, Evonik Goldschmidt GmbH) | 1,0 |
| NaOH (10%ige wässrige Lösung) | 0,6 |
| Parfüm, Konservierungsmittel | q.s. |
| Wasser dest. | ad 100 |
| | |
| **pH-Wert** | **7,2** |
| **SPF (Optometrics)** | **44** |

**Formulierungsbeispiel B:**

**O/W Sonnenschutzspray**

| **Zusammensetzung** | **%w/w** |
|---|---|
| **2-Phenoxyethyl-oktansäureester** | **5,6** |
| Decyl Cocoate (TEGOSOFT® DC, Evonik Goldschmidt GmbH) | 2,5 |
| Tocopheryl Acetate | 0,5 |
| Bis-Ethylhexyloxyphenol Methoxyphenyl Triazine | 3,0 |
| Ethylhexyl Triazone | 5,0 |
| Octocrylene | 5,0 |
| Butyl Methoxydibenzoylmethane | 3,0 |
| Cetearyl Glucoside (TEGO® Care CG 90, Evonik Goldschmidt GmbH) | 1,0 |
| Cetearyl Alcohol (TEGO® Alkanol 1618, Evonik Goldschmidt GmbH) | 0,5 |
| Glycerin | 2,0 |
| Carbomer (TEGO^{®} Carbomer 141, Evonik Goldschmidt GmbH)) | 0,05 |
| Acrylates/C10-30 Alkyl Acrylate Crosspolymer (TEGO^{®} Carbomer 341 ER, Evonik Goldschmidt GmbH) | 0,05 |
| Decyl Cocoate (TEGOSOFT® DC, Evonik Goldschmidt GmbH | 0,4 |
| NaOH (10%ige wässrige Lösung) | 0,4 |
| Parfüm, Konservierungsmittel | q.s. |
| Wasser dest. | ad 100 |
| | |
| **pH-Wert** | **7,4** |
| **SPF Optometrics** | **14** |

**Formulierungsbeispiel C:**

**O/W Sonnenschutzcreme**

| **Zusammensetzung** | **%w/w** |
|---|---|
| Cetearyl Alcohol (TEGO® Alkanol 1618, Evonik Goldschmidt GmbH) | 0,5 |
| Glyceryl Stearate (TEGIN® M Pellets, Evonik Goldschmidt GmbH) | 0,5 |
| Octocrylene | 5,0 |
| Ethylhexyl Salicylate | 5,0 |
| Bis-Ethylhexyloxyphenol Methoxyphenyl Triazine | 4,0 |
| Butyl Methoxydibenzoylmethane | 3,0 |
| Ethylhexyl Triazone | 2,0 |
| Ethylhexyl Methoxycinnamate | 3,0 |
| **2-Phenoxyethyl-oktansäureester** | **5,0** |
| Xanthan Gum | 0,3 |
| Acrylates/C10-30 Alkyl Acrylate Crosspolymer (TEGO^{®} Carbomer 341 ER, Evonik Goldschmidt GmbH) | 0,2 |
| Tocopheryl Acetate | 0,5 |
| Glyceryl Stearate Citrate (AXOL® C 62, Evonik Goldschmidt GmbH) | 2,5 |
| Glycerin | 3,0 |
| NaOH (10%ige wässrige Lösung) | 0,6 |
| Parfüm, Konservierungsmittel | q.s. |
| Wasser dest. | ad 100 |
| | |
| **pH-Wert** | **5,5** |
| **SPF Optometrics** | **50+** |

**Formulierungsbeispiel D:**

**leichte W/O Sonnenschutzlotion**

| **Zusammensetzung** | **%w/w** |
|---|---|
| Cetyl PEG/PPG-10/1 Dimethicone (ABIL® EM 90, Evonik Goldschmidt GmbH) | 2,5 |
| Diethylhexyl Carbonate (TEGOSOFT® DEC, Evonik Goldschmidt GmbH) | 3,5 |
| **2-Phenoxyethyl-oktansäureester** | **8,0** |
| Beeswax | 0,5 |
| Hydrogenated Castor Oil | 0,5 |
| Tocopheryl Acetate | 0,5 |
| Ethylhexyl Triazone | 3,0 |
| Ethylhexyl Methoxycinnamate | 3,0 |
| Ethylhexyl Salicylate | 4,0 |
| Butyl Methoxydibenzoylmethane | 4,0 |
| Bis-Ethylhexyloxyphenol Methoxyphenyl Triazine | 4,0 |
| Sodium Carboxymethyl Beta-Glucan (GluCare® S, Evonik Goldschmidt GmbH) | 0,2 |
| Allantoin | 0,1 |
| Glycerin | 1,0 |
| NaCl | 0,5 |
| Wasser, dest. | 64,7 |
| Parfüm, Konservierungsmittel | q.s. |
| | |
| **SPF Optometrics** | **50+** |

**Formulierungsbeispiel E:**

**W/O Sonnenschutzlotion**

| **Zusammensetzung** | **%w/w** |
|---|---|
| Polyglyceryl-4 Diisostearate/ Polyhydroxystearate/Sebacate (ISOLAN® GPS, Evonik Goldschmidt GmbH) | 3,0 |
| Hydrogenated Castor Oil | 0,2 |
| Microcrystalline Wax | 0,2 |
| Diethylhexyl Carbonate (TEGOSOFT® DEC, Evonik Goldschmidt GmbH) | 4,0 |
| **2-Phenoxyethyl-oktansäureester** | **8,0** |
| Octocrylene | 4,0 |
| Ethylhexyl Methoxycinnamate | 2,0 |
| Butyl Methoxydibenzoylmethane | 3,0 |
| Bis-Ethylhexyloxyphenol Methoxyphenyl Triazine | 4,0 |
| Ethylhexyl Triazone | 2,0 |
| Sodium Carboxymethyl Beta-Glucan (GluCare® S, Evonik Goldschmidt GmbH) | 0,1 |
| Sodium Hyaluronate (HyaCare®, Evonik Goldschmidt GmbH) | 0,1 |
| Glycerin | 2,0 |
| Wasser, dest. | 67,4 |
| Parfüm, Konservierungsmittel | q.s. |
| | |
| **SPF calculated** | **31** |

**Formulierungsbeispiel F:**

**Antiperspirant Stift**

| **Zusammensetzung** | **%w/w** |
|---|---|
| Cyclomethicone | 41,0 |
| Glyceryl Stearate (TEGIN® M Pellets, Evonik Goldschmidt GmbH) | 1,0 |
| Stearyl Alcohol (TEGO® Alkanol 18, Evonik Goldschmidt GmbH) | 18,0 |
| Hydrogenated Castor Oil | 2,5 |
| **2-Phenoxyethyl-oktansäureester** | **5,0** |
| C12-15 Alkyl Benzoate (TEGOSOFT® TN, Evonik Goldschmidt GmbH) | 4,0 |
| Diethylhexyl Carbonate (TEGOSOFT® DEC, Evonik Goldschmidt GmbH) | 5,0 |
| Aluminum Zirconium Tetrachloro-hydrex GLY | 20,0 |
| Talc | 3,0 |
| Silica | 0,5 |

**Formulierungsbeispiel G:**

**24 h AP/Deo Stift**

| **Zusammensetzung** | **%w/w** |
|---|---|
| PPG-3 Myristyl Ether (TEGOSOFT® APM, Evonik Goldschmidt GmbH) | 5,0 |
| **2-Phenoxyethyl-oktansäureester** | **5,0** |
| Stearyl Alcohol (TEGO® Alkanol 18, Evonik Goldschmidt GmbH) | 16,25 |
| Hydrogenated Castor Oil | 1,75 |
| Cyclomethicone | 44,5 |
| Silica Dimethyl Silylate (AEROSIL® R 972 V, Evonik Degussa GmbH) | 3,0 |
| Aluminum Chlorohydrate (50%) | 20,0 |
| Polyglyceryl-3 Caprylate (TEGO® Cosmo P 813, Evonik Goldschmidt GmbH) | 0,5 |
| Zinc Ricinoleate, Lysine, Propylene Glycol (TEGODEO® LYS, Evonik Goldschmidt GmbH) | 4,0 |

**Formulierungsbeispiel H:**

**PEG-freie O/W Antiperspirant Lotion**

| **Zusammensetzung** | **%w/w** |
|---|---|
| Methyl Glucose Sesquistearate (TEGO® Care PS, Evonik Goldschmidt GmbH | 1,75 |
| Polyglyceryl-4 Laurate (TEGO® Care PL 4, Evonik Goldschmidt GmbH) | 0,25 |
| **2-Phenoxyethyl-oktansäureester** | **3,0** |
| Diethylhexyl Carbonate (TEGOSOFT® DEC, Evonik Goldschmidt GmbH) | 2,0 |
| PPG-14 Butyl Ether (TEGOSOFT® PBE, Evonik Goldschmidt GmbH) | 2,0 |
| Polyglyceryl-3 Caprylate (TEGO® Cosmo P 813, Evonik Goldschmidt GmbH) | 0,5 |
| Wasser, dest. | 74,5 |
| Hydroxyethyl Cellulose | 1,0 |
| Aluminum Chlorohydrate (50%) | 15,0 |
| Parfüm | q.s. |

**Formulierungsbeispiel J:**

**desodorierende O/W Hand- und Fußcreme**

| **Zusammensetzung** | **%w/w** |
|---|---|
| Ceteareth-25 (TEGINACID® C, Evonik Goldschmidt GmbH) | 2,0 |
| Glyceryl Stearate (TEGIN® M Pellets, Evonik Goldschmidt GmbH) | 4,0 |
| Stearyl Alcohol (TEGO® Alkanol 18, Evonik Goldschmidt GmbH) | 2,5 |
| Cetearyl Isononanoate (TEGOSOFT® CI, Evonik Goldschmidt GmbH) | 2,5 |
| **2-Phenoxyethyl-oktansäureester** | **4,0** |
| Zinc Ricinoleate (TEGODEO® PY 88, Evonik Goldschmidt GmbH) | 2,0 |
| Glycerin | 3,0 |
| Wasser, dest. | 79,0 |
| Carbomer (TEGO® Carbomer 134, Evonik Goldschmidt GmbH) | 0,2 |
| Cetearyl Isononanoate (TEGOSOFT® CI, Evonik Goldschmidt GmbH) | 0,8 |

**Formulierungsbeispiel K:**

**O/W Antiperspirant Roll-On**

| **Zusammensetzung** | **%w/w** |
|---|---|
| Steareth-2 (TEGO® Alkanol S 2 Pellets, Evonik Goldschmidt GmbH) | 2,2 |
| Steareth-20 (TEGO® Alkanol S 20 P, Evonik Goldschmidt GmbH) | 1,0 |
| Cetearyl Ethylhexanoate (TEGOSOFT® liquid, Evonik Goldschmidt GmbH) | 2,0 |
| **2-Phenoxyethyl-oktansäureester** | **2,0** |
| Dimethicone (ABIL® 350, Evonik Goldschmidt GmbH) | 0,5 |
| Glycerin | 3,0 |
| Wasser, dest. | 58,8 |
| Triethylcitrate | 0,5 |
| Aluminum Chlorohydrate (50%) | 30,0 |

## Patentansprüche

1. Verwendung von Phenoxyalkylestern der allgemeinen
Formel (I) 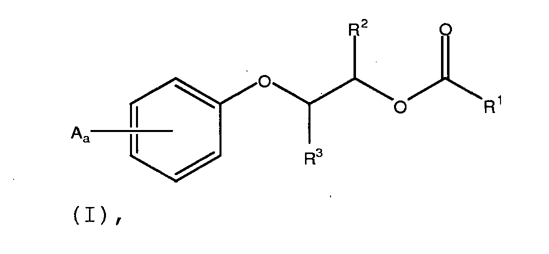
wobei die Gruppe OOC-R¹ der Säurerest der Säuren ausgewählt aus der Gruppe:
Cyclohexancarbonsäure, Caprylsäure, 2-Ethylhexansäure, Isononansäure, 3,5,5-Trimethylhexansäure, 2-Propylheptansäure und
2-Butyloktansäure, so wie Mischungen enthaltend
mindestens zwei dieser Carbonsäuren ist,
R² und R³ unabhängig voneinander, gleich oder verschieden, Wasserstoff oder Methyl und
Aₐ mit a=1-5 unabhängig voneinander, gleich oder verschieden ausgewählt aus der Gruppe: H, F, Cl, Alkyl- oder Fluoralkylrest, CN, CO₂R⁴, OH, OR⁵, O₂CR⁶, NR⁷R⁸, NO₂, SO₃R⁹, mit R⁴ bis R⁹ unabhängig voneinander, gleich oder verschieden ausgewählt aus der Gruppe: H, Alkyl- oder Fluoralkylrest,
sind,
zur Herstellung einer kosmetischen, dermatologischen oder pharmazeutischen Formulierung, **dadurch gekennzeichnet, dass** die Formulierung ein Sonnenschutzpräparat ist.

2. Verwendung gemäß Anspruch 1 **dadurch gekennzeichnet, dass** R² und R³ ausgewählt sind aus der Gruppe:
R² = R³ = H,
R² = H, R³ = Methyl,
R² = Methyl, R³ = H,
so wie Kombinationen dieser.

3. Verwendung gemäß mindestens einem der Ansprüche 1 oder 2, **dadurch gekennzeichnet, dass** es sich bei den Phenoxyalkylestern um einen 2-Phenoxyethyloktansäureester, 2-Phenoxyethyl-2-ethylhexansäureester, 2-Phenoxyethylcyclohexancarbonsäureester, 2-Phenoxyethylisononansäureester, 2-Phenoxyethyl-3,5,5-trimethylhexansäureester, 2-Phenoxyethyl-2-butyloktansäureester, 1-Phenoxy-2-propyloktansäureester, 1-Phenoxy-2-propyl-2-ethylhexansäureester, 1-Phenoxy-2-propylcyclohexancarbonsäureester, 1-Phenoxy-2-propylisononansäureester, 1-Phenoxy-2-propyl-3,5,5-trimethylhexansäureester oder 1-Phenoxy-2-propyl-2-butyloktansäureester handelt.

4. Verwendung mindestens einer Substanz der Formel (I) wie in mindestens einem der Ansprüche 1 bis 3 beschrieben als Lösungsvermittler oder Lösungsmittel für mindestens eine lipophile, hydrophobe UV-Lichtschutzfiltersubstanz, **dadurch gekennzeichnet, dass** mindestens eine der UV-Lichtschutzfiltersubstanzen ein Triazinderivat ist, oder ausgewählt ist aus der Gruppe der Verbindungen, enthaltend 2-Cyano-3-phenyl-zimtsäure-2-ethylhexylester, 2,4-Bis-{[4-(2-ethyl-hexyloxy)-2-hydroxy]-phenyl}-6-(4-methoxyphenyl)-1,3,5-triazin,
Dioctylbutylamidotriazon,
2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Tris-[anilino-(p-carbo-2'-ethyl-1'-hexyloxy)]-1,3,5-triazin, 2,4-bis-[5,1(dimethylpropyl)benzoxazol-2-yl-(4-phenyl)-imino]-6-(2-ethylhexyl).-imino-1,3,5-triazin, 2,4-Bis-{[4-(2-Ethyl-hexyloxy)-2-hydroxy]-phenyl}-6-(4-methoxyphenyl)-1,3,5-triazin und 2-[4,6-Bis(2,4-dimethylphenyl)-1,3,5-triazin-2-yl]-5-(octyloxy)phenol.

5. Kosmetische, dermatologische oder pharmazeutische Formulierung enthaltend mindestens eine Substanz der allgemeinen Formel (I) wie in mindestens einem der Ansprüche 1 bis 3 beschrieben, **dadurch gekennzeichnet, dass** sie
(a) 3 - 60 Gewichtsprozent mindestens einer Substanz der allgemeinen Formel (I),
(b) 0,1 - 20 Gewichtsprozent Tenside und/oder Emulgatoren und/oder Coemulgatoren,
(c) 0,1 - 40 Gewichtsprozent weiterer Ölkörper
(d) 0 - 98 Gewichtsprozent Wasser
enthält,
wobei die Gewichtsprozente der Komponenten (a), (b), (c) und (d) sich auf 100 Gewichtsprozent addieren.

## Claims

1. Use of phenoxyalkyl esters of the general formula (I) 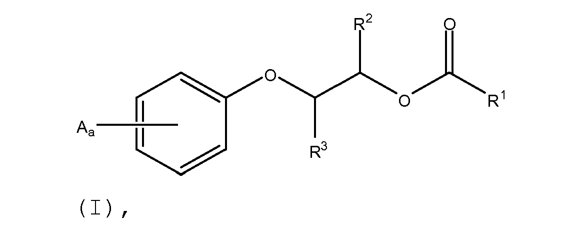 where the group OOC-R¹ of the acid radical of the acids is selected from the group:
cyclohexanecarboxylic acid, caprylic acid, 2-ethylhexanoic acid, isononanoic acid, 3,5,5-trimethylhexanoic acid, 2-propylheptanoic acid and 2-butyloctanoic acid, and mixtures comprising at least two of these carboxylic
acids,
R² and R³, independently of one another, identical or different, are hydrogen or methyl and
Aₐ where a=1-5, independently of one another, identical or different, are selected from the group: H, F, Cl, alkyl or fluoroalkyl radical, CN, CO₂R⁴, OH, OR⁵, O₂CR⁶, NR⁷R⁸, NO₂, SO₃R⁹, where R⁴ to R⁹, independently of one another, identical or different, are selected from the group: H, alkyl or fluoroalkyl radical,
for the preparation of a cosmetic, dermatological or pharmaceutical formulation, **characterized in that** the formulation is a sunscreen preparation.

2. Use according to Claim 1, **characterized in that** R² and R³ are selected from the group:
R² = R³ = H,
R² = H, R³ = methyl,
R² = methyl, R³ = H,
and combinations of these.

3. Use according to at least one of Claims 1 and 2, **characterized in that** the phenoxyalkyl esters are a 2-phenoxyethyloctanoic acid ester, 2-phenoxyethyl-2-ethylhexanoic acid ester, 2-phenoxyethylcyclohexancarboxylic acid ester, 2-phenoxyethylisononanoic acid ester, 2-phenoxyethyl-3,5,5-trimethylhexanoic acid ester, 2-phenoxyethyl-2-butyloctanoic acid ester, 1-phenoxy-2-propyloctanoic acid ester, 1-phenoxy-2-propylpelargonic acid ester, 1-phenoxy-2-propylcyclohexanecarboxylic acid ester, 1-phenoxy-2-propylisononanoic acid ester, 1-phenoxy-2-propyl-3,5,5-trimethylhexanoic acid ester or 1-phenoxy-2-propyl-2-butyloctanoic acid ester.

4. Use of at least one substance of the formula (I) as described in at least one of Claims 1 to 3 as solubility promoter or solvent for at least one lipophilic, hydrophobic UV photoprotective filter substance, **characterized in that** at least one of the UV photoprotective filter substances is a triazine derivative or is selected from the group of compounds comprising
2-ethylhexyl 2-cyano-3-phenylcinnamate, 2,4-bis{[4-(2-ethylhexyloxy)-2-hydroxy]phenyl}-6-(4-methoxyphenyl)-1,3,5-triazine, dioctylbutylamidotriazone, 2-hydroxy-4-methoxybenzophenone, 2-hydroxy-4-methoxy-4'-methylbenzophenone, 2,2'-dihydroxy-4-methoxybenzophenone, di-2-ethylhexyl 4-methoxybenzmalonate, 2,4,6-tris[anilino(p-carbo-2'-ethyl-1'-hexyloxy)]-1,3,5-triazine, 2,4-bis[5,1-(dimethylpropyl)benzoxazol-2-yl-(4-phenyl)-imino]-6-(2-ethylhexyl)-imino-1,3,5-triazine, 2,4-bis{[4-(2-ethylhexyloxy)-2-hydroxy]phenyl}-6-(4-methoxyphenyl)-1,3,5-triazine and 2-[4,6-bis(2,4-dimethylphenyl)-1,3,5-triazin-2-yl]-5-(octyloxy)phenol.

5. Cosmetic, dermatological or pharmaceutical formulation comprising at least one substance of the general formula (I) as described in at least one of Claims 1 to 3, **characterized in that** it comprises
(a) 3 - 60 per cent by weight of at least one substance of the general formula (I),
(b) 0.1 - 20 per cent by weight of surfactants and/or emulsifiers and/or coemulsifiers,
(c) 0.1 - 40 per cent by weight of further oil bodies
(d) 0 - 98 per cent by weight of water,
where the percentages by weight of components (a), (b), (c) and (d) add up to 100 per cent by weight.

## Revendications

1. Utilisation d'esters phénoxyalkyliques de formule générale (I) 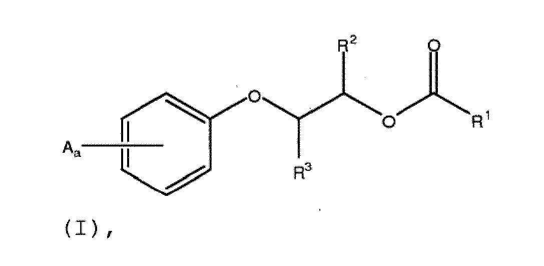 dans laquelle le groupe OOC-R¹ est le radical acide des acides choisis dans le groupe constitué par :
l'acide cyclohexanecarboxylique, l'acide caprylique,
l'acide 2-éthylhexanoïque, l'acide isononanoïque,
l'acide 3,5,5-triméthylhexanoïque, l'acide 2-propylheptanoïque et l'acide 2-butyloctanoïque, ainsi que les mélanges contenant au moins deux de ces acides carboxyliques,
R² et R³ représentent indépendamment l'un de l'autre, de manière identique ou différente, hydrogène ou méthyle,
et
les Aₐ, avec a = 1 à 5, sont choisis indépendamment les uns des autres, de manière identique ou différente,
dans le groupe constitué par : H, F, Cl, radical alkyle ou fluoroalkyle, CN, CO₂R⁴, OH, OR⁵, O₂CR⁶, NR⁷R⁸, NO₂, SO₃R⁹, avec R⁴ à R⁹ choisis indépendamment les uns des autres, de manière identique ou différente, dans le groupe constitué par : H, radical alkyle ou fluoroalkyle,
pour la fabrication d'une formulation cosmétique,
dermatologique ou pharmaceutique, **caractérisée en ce que** la formulation est une préparation de protection solaire.

2. Utilisation selon la revendication 1, **caractérisée en ce que** R² et R³ sont choisis dans le groupe :
R² = R³ =H,
R² = H, R³ = méthyle,
R² = méthyle, R³ = H,
ainsi que leurs combinaisons.

3. Utilisation selon au moins l'une quelconque des revendications 1 ou 2, **caractérisée en ce que** les esters phénoxyalkyliques sont un ester de l'acide 2-phénoxyéthyloctanoïque, un ester de l'acide 2-phénoxyéthyl-2-éthylhexanoïque, un ester de l'acide 2-phénoxyéthylcyclohexanecarboxylique, un ester de l'acide 2-phénoxyéthylisononanoïque, un ester de l'acide 2-phénoxyéthyl-3,5,5-triméthylhexanoïque, un ester de l'acide 2-phénoxyéthyl-2-butyloctanoïque, un ester de l'acide 1-phénoxy-2-propyloctanoïque, un ester de l'acide 1-phénoxy-2-propyl-2-éthylhexanoïque, un ester de l'acide 1-phénoxy-2-propylcyclohexanoïque, un ester de l'acide 1-phénoxy-2-propylisononanoïque, un ester de l'acide 1-phénoxy-2-propyl-3,5,5-triméthylhexanoïque ou un ester de l'acide 1-phénoxy-2-propyl-2-butyloctanoïque.

4. Utilisation d'au moins une substance de formule (I) telle que décrite dans au moins l'une quelconque des revendications 1 à 3 en tant que solubilisant ou solvant pour au moins une substance filtre protecteur contre la lumière UV lipophile et hydrophobe, **caractérisée en ce qu'**au moins une des substances filtre protecteur contre la lumière UV est un dérivé de triazine ou est choisie dans le groupe des composés contenant :
l'ester 2-éthylhexylique de l'acide 2-cyano-3-phénylcinnamique,
la 2,4-bis-{[4-(2-éthyl-hexyloxy)-2-hydroxy]-phényl}-6-(4-méthoxyphényl)-1,3,5-triazine,
la dioctylbutylamidotriazone,
la 2-hydroxy-4-méthoxybenzophénone,
la 2-hydroxy-4-méthoxy-4'-méthylbenzophénone,
la 2,2'-dihydroxy-4-méthoxybenzophénone,
l'ester di-2-éthylhexylique de l'acide 4-méthoxybenzmalonique,
la 2,4,6-tris-[anilino-(p-carbo-2'-éthyl-1'-hexyloxy)]-1,3,5-triazine,
la 2,4-bis-[5,1(diméthylpropyl)benzoxazol-2-yl-(4-phényl)-imino]-6-(2-éthylhexyl)-imino-1,3,5-triazine,
la 2,4-bis-{[4-(2-éthyl-hexyloxy)-2-hydroxy]-phényl}-6-(4-méthoxyphényl)-1,3,5-triazine et
le 2-[4,6-bis(2,4-diméthylphényl)-1,3,5-triazin-2-yl]-5-(octyloxy)phénol.

5. Formulation cosmétique, dermatologique ou pharmaceutique contenant au moins une substance de formule générale (I) telle que décrite dans au moins l'une quelconque des revendications 1 à 3, **caractérisée en ce qu'**elle continent :
(a) 3 à 60 pour cent en poids d' au moins une substance de formule générale (I),
(b) 0,1 à 20 pour cent en poids de tensioactifs et/ou d'émulsifiants et/ou de co-émulsifiants,
(c) 0,1 à 40 pour cent en poids d'autres corps huileux,
(d) 0 à 98 pour cent en poids d'eau,
les pourcentages en poids des composants (a), (b), (c) et (d) s'additionnant pour atteindre 100 pour cent en poids.
